# FASCICULE DE BREVET EUROPEEN

(11) **EP 1 753 838 B1**
(45) Date de publication et mention de la délivrance du brevet: **14.03.2012**
(21) Numéro de dépôt: 05773208.3
(22) Date de dépôt: 20.05.2005
(51) Int. Cl.: C07D 311/92, C09K 9/02, G02B 5/23

(54) **DERIVES DE NAPHTOPYRANES POLYMERISABLES ET MATERIAUX POLYMERES OBTENUS A PARTIR DE CES DERIVES**
POLYMERISIERBARE NAPHTHOPYRANDERIVATE UND AUS DERARTIGEN DERIVATEN GEWONNENE POLYMERMATERIALIEN
POLYMERISABLE NAPHTHOPYRAN DERIVATIVES AND POLYMER MATERIALS OBTAINED FROM SAID DERIVATIVES

(30) Priorité: 21.05.2004 FR 0405515
(43) Date de publication de la demande: 21.02.2007
(73) Titulaire: Polymerexpert S.A., 33600 Pessac (FR)
(72) Inventeur: GIBANEL, Sébastien, Pittsburgh, PA 15222 (US); POZZO, Jean-Luc, F-33470 GUJAN-MESTRAS (FR); PAGNOUX, Anne, F-33114 LE BARP (FR); DOLATKHANI, Marc, F-33610 CESTAS (FR)
(74) Mandataire: Ulmann, Catherine Claire
(86) Numéro de dépôt international: PCT/FR2005/001266
(87) Numéro de publication internationale: WO 2005/123872

(56) Documents cités:
- EP-A- 1 038 870
- WO-A1-01/30866
- US-A- 4 929 693
- US-A- 6 113 814
- US-A1- 2001 025 948

## Description

L'invention concerne de nouveaux dérivés de naphtopyranes polymérisables ainsi que des matériaux polymères obtenus à partir de ces dérivés.

Les matériaux photochromiques sont des matériaux bien connus pour changer de couleur réversiblement lorsqu'ils sont exposés à la lumière, par exemple aux rayons ultraviolets. De nombreuses applications dans le domaine de l'optique ophtalmique ont été développées autour de ces molécules : le brunissement des verres de lunettes au soleil, le changement de couleur de lentilles de contact, etc... permettent de protéger la rétine de l'agression des rayons ultraviolets.

Les agents photochromiques les plus utilisés sont :
- les spiro-indolino-pyranes : la coloration développée est très intense sous peu de rayonnement pour les composés substitués par l'un ou plusieurs des groupements -nitro, -cyano, amino, alcoxy. Cependant les passages fréquents entre forme stable et forme excitée provoquent rapidement la dégradation de la molécule.
- les spirobenzothiazolo-benzopyranes et/ou spiroindolino-benzothiopyranes : La coloration développée sous irradiation est dans la gamme des bleus, mais les vitesses de décoloration thermique sont lentes par rapport aux naphtopyranes et la faible efficacité de coloration reste un inconvénient majeur à l'emploi de ces substances comme pigment photochromique.
- les spiro-indolino-oxazines : Ces composés ont été largement utilisés dans le domaine des matériaux à transmission optique variable et ont montré des propriétés d'ensemble remarquables : coloration, résistance à la fatigue, vitesse de décoloration thermique compatibles avec les applications recherchées. Toutefois il est à noter que ces propriétés sont restreintes à des molécules développant une coloration allant du bleu au vert. Les modifications chimiques permettant d'atteindre la couleur rouge entraîne une très forte augmentation de la photofatigue.
- les naphtopyranes : Parmi les naphtopyranes, les composés de type 3,3-diaryl-3H-naphto[2,1-b]pyranes présentent une intense coloration dans le domaine du jaune-orange-rouge, une résistance à la fatigue exaltée en présence des substituants phényle en position 3 et des cinétiques de décoloration thermique en absence d'irradiation lumineuse compatible avec les applications recherchées par les inventeurs de la présente invention. On peut obtenir d'autres couleurs avec d'autres substituants, à certaines températures (US3567605, 1971). Ce type de molécule est un bon compromis du point de vue de ses performances photochromiques : une coloration et décoloration rapide dans une gamme de température d'utilisation large (0 à 40°C), une coloration intense sous forme excitée (US5631720, JP8176139, JP8157467, US6113814, WO97/05213). De nouvelles molécules dérivées, comme les tétraphényl naphtodipyranes (US5464567) ou les indéno-anellé naphtopyranes (W09614596) ont été développées pour étendre le spectre émis de l'orange au bleu/gris.

Les naphtopyranes sont un bon compromis entre intensité de la couleur et vitesse de décoloration. De plus, leur changement de structure sous forme excitée n'est pas perturbé par la matrice polymère environnante et ils présentent une bonne résistance à la fatigue.

On rappelle ci-après le principe de coloration sous l'action des UV : le cycle porteur de l'atome d'oxygène s'ouvre et la conjugaison des doubles liaisons qui en résulte entraîne le développement de colorations.

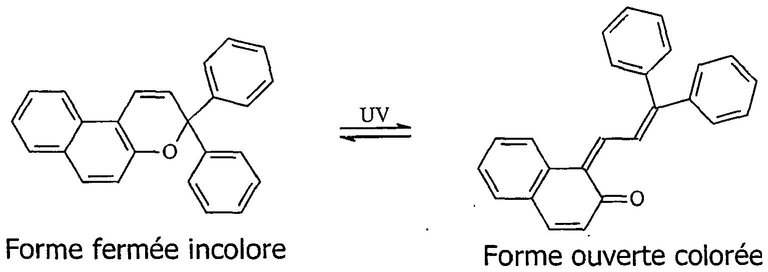

Les matériaux photochromiques sont généralement fabriqués à partir de matrices polymères au sein desquelles les molécules photochromes sont dispersées (WO0160811). Depuis 1990, certains auteurs ont fonctionnalisé ces molécules pour les rendre polymérisables :
- Toray Industries a développé une spirooxazine polymérisable en 1990 (US5166345), Nat Science Council en a développé un autre en 1997 (US582187)
- Otsuka Kagaku s'est intéressé aux performances des spiropyranes polymérisables en 1992-1993 (US5236958, US5252742), Mr Yun Ki également (US2003099910)
- Sola International Holdings a intercalé un espaceur entre le photochrome et la fonction polymérisable pour le rendre plus compatible avec la matrice (W09705213)
- Transitions Optical décrit dans WO03056390 et US6113814 toutes les techniques connues à ce jour pour rendre un photochrome polymérisable, mais il ne décrit que des molécules de la famille des naphtopyranes portant un substituant diméthoxyphényle sur le carbone n°3 ou un groupement polymérisable lié au naphtalène via au moins un motif O-CH₂-CH(H ou CH₃)-O).

Le principe de fonctionnalisation de molécules photochromes pour les rendre copolymérisables avec les monomères servant à synthétiser la matrice polymère n'est pas nouveau. Ainsi, les molécules photochromes sont liées chimiquement à la matrice polymère et elles ne peuvent plus migrer dans le temps, en fonction de la température à laquelle le matériau photochrome est soumis.

Les inventeurs de la présente invention ont maintenant mis au point de nouvelles molécules dérivées des 3;3-diaryl-3H-naphto[2,1-b]pyranes (naphtopyranes) permettant des applications pour lesquelles ces molécules photochromes sont en contact avec le corps humain, en particulier en dermatologie, en cosmétologie et en ophtalmologie et dont la toxicité est très réduite voire même inexistante, du fait que ces molécules ne sont pas assimilables par les cellules.

Par ailleurs, ces molécules ont pu être préparées de façon nettement simplifiées par rapport aux synthèses connues dans l'art antérieur, notamment en évitant des étapes intermédiaires de purification particulièrement délicates et onéreuses, ce qui confère à ces nouveaux produits un caractère industriel en évitant en particulier les étapes de purification des intermédiaires de synthèse obtenus, comme c'est par exemple le cas, selon la publication Pozzo et al., Langmuir (2002), 18(19), 7096-7101.

Ainsi, les nouvelles molécules photochromes polymérisables de la présente invention présentent une bonne coloration dans le jaune-orange, une vitesse de décoloration rapide et une résistance à la fatigue élevée et peuvent en outre être aisément préparées industriellement.

Plus précisément, le photochrome de l'invention est un monomère au moins divalent (co)polymérisable par polymérisation en chaîne ou par étapes. Il s'agit d'un 3,3-diaryl-3H-naphto[2,1-b]pyrane substitué qui présente, comme indiqué précédemment, et, comme cela ressort de la description et des exemples, l'avantage de pouvoir être aisément synthétisé à l'échelle industrielle.

En raison de son plus faible coût de production et du relativement bon rendement de synthèse, on peut envisager des applications de cette molécule en optique, ophtalmologie, en cosmétique, voire dans des secteurs tels que le textile, le bâtiment, etc. De plus, comme elle est copolymérisable avec les produits entrant dans leur composition, sa stabilité sera d'autant améliorée dans les matériaux correspondants.

Ainsi, ces molécules photochromiques peuvent être copolymérisées avec tous les monomères existants, seules ou en mélange avec d'autres photochromes polymérisables décrits dans la littérature.

L'invention concerne donc, selon un premier aspect, une nouvelle famille de dérivés de naphtopyranes polymérisables.

Selon un deuxième aspect, l'invention concerne le procédé de synthèse de ces nouveaux composés.

Selon un troisième aspect, l'invention concerne des produits nouveaux résultant de la polymérisation ou de la copolymérisation des naphtopyranes de l'invention ainsi que des produits obtenus par modification chimique d'un polymère ou d'un oligomère par réaction avec un naphtopyrane de l'invention. L'invention trouve des applications dans différents domaines où l'on cherche des propriétés de filtration des ultraviolets et/ou de photochromisme.

Plus précisément, selon la caractéristique essentielle de son premier aspect, l'invention concerne un composé du type 3,3-diaryl-3H-naphto[2,1-b]pyrane substitué polymérisable caractérisé en ce qu'il répond à la formule (I) : 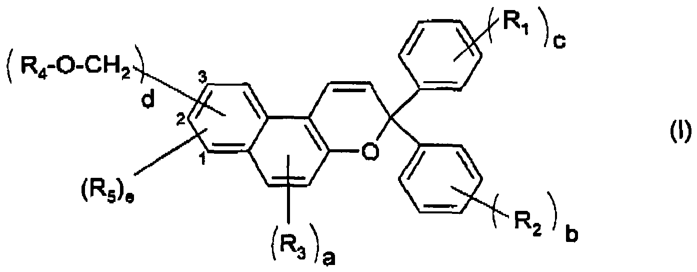 dans laquelle
• R₁, R₂ et R₃ sont tels qu'ils sont identiques ou différents et représentent indépendamment :
   - un hydrogène,
   - un halogène,
   - un groupement hydroxy ou hydroxyalkyle en C1 à C15 ou
   - un groupement alkyle linéaire ou ramifié en C1 à C15 a, b et c étant indépendamment compris entre 0 et 5 ;
• R₄ est lié au motif naphtalène en position 1, 2 ou 3 via une liaison -CH₂-O- et choisi parmi :
   - les groupes divalents suivants :
      CH₂=CH-C(=O)-, CH₂=C(CH₃)-C(=O)-, -(CH₂)-OC(=O)CH=CH₂, -(CH₂)-OC(=O)C(CH₃)=CH₂, -(CH₂)_{n'}-CH=CH₂, -(CH₂)-O-(CH)_{n'}-CH=CH₂, -(CH₂)-O-CH=CH₂, n'étant compris entre 0 et 15,
      et, dans ce cas, le(s) groupe(s) R₅ est (sont) H, (CₙH₂ₙ)-CH₃, (CₙH₂ₙ)-OH, n étant compris entre 1 et 15, et
      d est alors compris entre 1 et 3
   - les groupes monovalents suivants :
      l'hydrogène, (CH₂)-NH₂,-(CₚH₂ₚ) - [CH-CH₂-O]cycle,
      -(CH₂)-COOH, -(CH₂)Si(CₘH₂ₘ)₂-H, -(CH₂)-Si-CH=CH₂, -(CH₂)Si(O-CₘH₂ₘ)₃,
      -C(=O)NH-R-N=C=O, avec R = (CₙH₂ₙ), (CₙH₂ₙ₋₂), aryle en C₅ à C₂₀,
      (CₙH₂ₙ₋₂)-CH₂(CₙH₂ₙ₋₂) et Aryle-CH₂-Aryle, n et m étant compris entre 1 et 15 et
      p étant compris entre 0 et 15,
      et dans ce cas le groupe R₅, s'il y en a un, est H ou un alkyle en C₁-C₁₅, et
      d est alors égal à 2 ou 3 ;
• R₅ est tel que défini ci-dessus et est lié au motif naphtalène en position 1, 2 ou 3
et e est un entier compris entre 0 et 2 et tel que d+ e = 3.

Ainsi, ce qui caractérise les composés de l'invention et les distingue de composés existants dans l'art antérieur est la présence sur le groupement naphténique de substituants réactifs, liés au cycle aromatique par une liaison -CH₂-O-, permettant à ces composés de participer à des réactions de (co)polymérlsations en chaîne ou par étape. Ceci est rendu possible par le fait que les groupes R₄ sont tels que l'un au moins est un groupe divalent (co)polimérisable avec un monomère ou que deux au moins de ces groupes comprennent un groupe monovalent (co)polymérisable avec un monomère.

Dans le cas des groupes polymérisables divalents, l'incorporation puis l'ancrage du photochrome dans la matière se fera par polymérisation en chaîne (anionique, cationique, radicalaire, ouverture de cycle, métathèse).

Dans le cas des groupes polymérisables monovalents, la synthèse du matériau photochrome se fera par polymérisation par étapes entre monomères ou oligomères difonctionnels, c'est à dire portant au moins deux substituants monovalents. On formera alors par polycondensation ou polyaddition des polyuréthanes, des polyesters, des polyéthers, des polyamides, des polysiloxanes, etc... Les autres groupes portés par le motif naphtalène (groupes désignés par R₅) sont des groupes inertes et devront donc être non réactifs vis-à-vis du mécanisme de polymérisation par étapes et sera choisi parmi l'hydrogène, les alkyles ou les halogènes.

Les groupements R₁ et R₂ sont avantageusement choisis indépendamment dans le groupe constitué de l'hydrogène, du méthyle, du méthoxy et du fluor et R₃ et R₅ sont avantageusement hydrogène.

On comprendra aisément qu'en fonction de la nature des fonctions polymérisables portées par le cycle naphténique, les monomères de l'invention pourront être impliqués dans des réactions de polymérisatlon en chaîne ou de polymérisation par étape.

Plus précisément, le monomère de l'invention pourra être impliqué dans une réaction de polymérisation en chaîne soit lorsqu'il est sous la forme d'un monomère divalent copolymérisable, soit lorsqu'il est sous la forme d'un monomère tétravalent copolymérisable qui peut alors servir d'agent de réticulation.

Un exemple de cas où le monomère de l'invention se comporte comme un monomère divalent copolymérisable est celui où l'un des trois groupes liés aux positions 1,2 ou 3 du cycle naphténique est constitué du motif R₄-O-CH₂- dans lequel R4 est un groupe divalent tel que défini plus haut.

Un exemple de cas où le monomère de l'invention se comporte comme un monomère tétravalent copolymérisable est celui où deux des groupes liés aux positions 1,2 ou 3 du cycle naphténique sont constitués du motif R₄-O-CH₂- dans lequel R₄ est un groupe divalent tel que défini plus haut.

Par ailleurs, les monomères de l'invention pourront être impliqués dans des réactions de polymérisation par étape lorsqu ils sont en particulier sous la forme de monomères divalents (co)polymérisables, c'est à dire qu'ils portent 2 groupes monovalents, ou de monomères trivalents copolymérisables pouvant servir d'agent de réticulation.

Un exemple de tels monomères divalents (co)polymérisables est celui des monomères de l'invention dans lesquels deux des groupes liés au motif naphtalène en position 1, 2 ou 3 sont constitués du motif R₄-O-CH₂-dans lequel R₄ est un groupe monovalent tel que défini plus haut

Un exemple de monomère trivalent copolymérisable est celui dans lequel les trois groupes R₄ sont choisis indépendamment parmi les groupes monovalents tels que définis plus haut.

A titre de monomères préférés de l'invention comprenant au moins deux groupements polymérisables monovalents, on citera ceux dans lesquels au moins deux des groupes R₄ sont choisis parmi les groupes monovalents tels que définis plus haut.

Parmi ces composés, ceux comprenant trois groupements R₄ choisis indépendamment dans le groupe tel que défini ci-dessus permettent d'accéder à des monomères trivalents copolymérisables pouvant servir d'agent de réticulation.

En ce qui concerne les monomères de l'invention comprenant deux groupes R₄ polymérisables monovalents, il est à noter que les fonctions polymérisables peuvent être soit de même nature, soit de nature différente, par exemple :
- un groupe porteur d'un acide carboxylique et un groupe amine, le groupe R₅ étant soit un hydrogène, soit un alkyle.

Selon un deuxième aspect, l'invention concerné également un procédé de fabrication des monomères qui font l'objet du premier aspect de l'invention.

L'homme du métier comprendra aisément que, du fait, de la grande variété des substituants R₄, il soit difficile de présenter un schéma général de synthèse de l'ensemble des produits de l'invention.

Toutefois, le point commun de l'ensemble des schémas de synthèse envisagés est le fait qu'ils présentent l'avantage de comprendre une étape de cyclisation que l'on désignera ci-après par étape de chroménisation, au cours de laquelle on réalise la précipitation d'un produit intermédiaire répondant à la formule il ci-dessous : 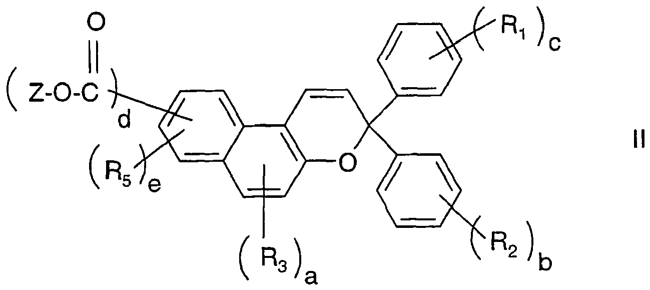 dans laquelle le groupement Z est soit l'hydrogène, soit un groupement alkyle CₙH₂ₙ₊₁ avec n= 1 à 15, soit un précurseur, éventuellement protégé des groupements R₄, les groupements R₁, R₂, R₃, R₄ et R₅ ainsi que a, b, c, d et e étant tels que définis précédemment.

Ce produit précipité subira ensuite avantageusement une étape de réduction qui conduira à la formation de la liaison CH₂O qui relie le ou les groupements polymérisables au cycle naphténique et qui constitue une des caractéristiques essentielles des produits de l'invention. En effet, une étape clé est le choix du bon couple solvant - non solvant permettant la précipitation du produit présentant la fonction ester. L'homme du métier comprend, bien entendu, que la nature de ce couple peut varier en fonction de la nature des substituants, celui utilisé dans les schémas de synthèse donnés ci-après convenant particulièrement bien à la synthèse des produits décrits.

La précipitation de cet intermédiaire présente l'avantage de permettre l'obtention d'un produit de grande pureté sans avoir recours aux étapes de purification nécessaires selon l'art antérieur, ce qui représente un avantage industriel particulièrement considérable.

On donne ci-dessous le schéma de synthèse complet dans le cas des monomères préférés de l'invention dans lequel le groupe R₄ est un groupe polymérisable divalent de type (méth)acrylique :

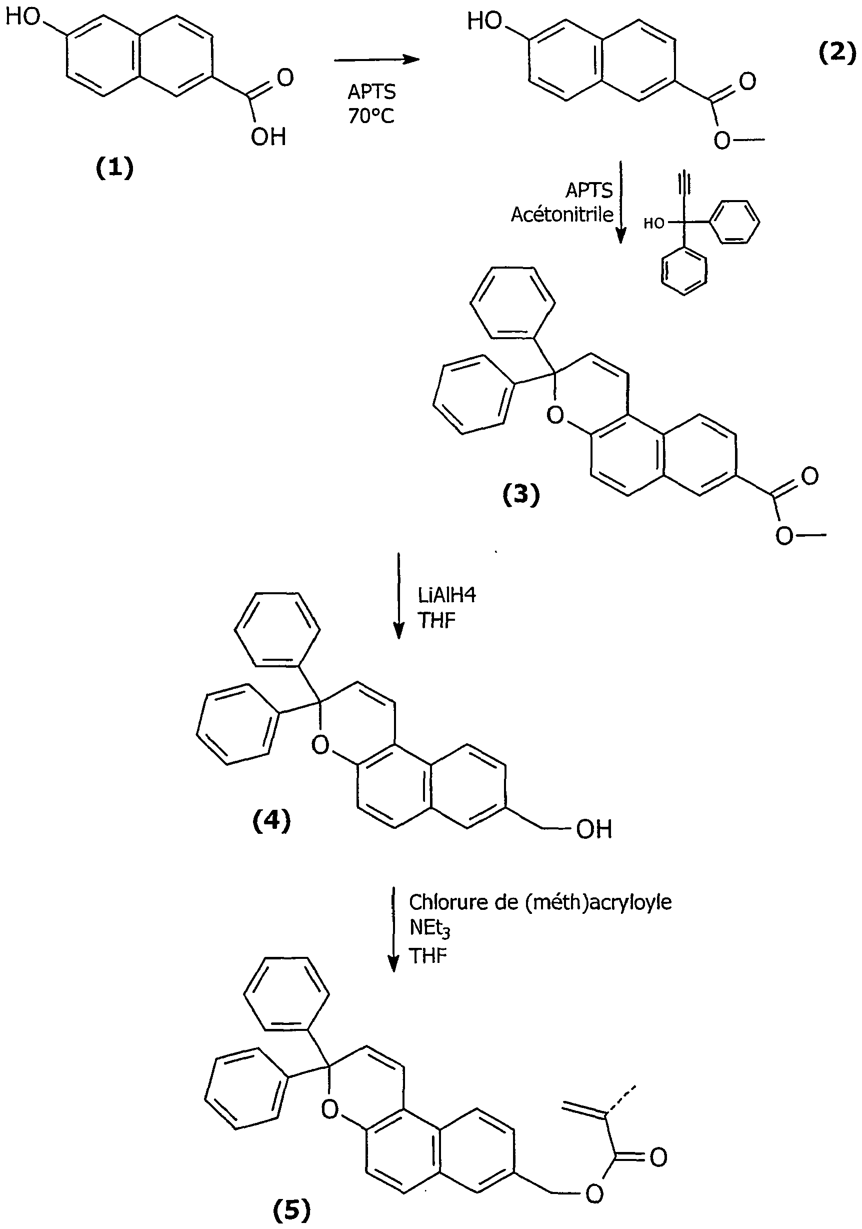

Comme cela apparaît sur le schéma de synthèse ci-dessus qui fait l'objet d'une description détaillée dans les exemples 1 et 2 qui suivent, le produit final (produit 5) est obtenu dans des étapes parfaitement industrialisables du fait du bon rendement de chacune de ces étapes et de la pureté des produits obtenus.

On notera notamment que le produit (3) peut être isolé de façon particulièrement simple et efficace par une simple filtration du fait du choix du milieu réactionnel dans lequel est réalisé la cyclisation (chroménisation) permettant de le synthétiser à partir du composé (2) de l'étape précédente.

Cette étape de cyclisation au cours de laquelle on précipite l'intermédiaire recherché pour les étapes ultérieures s'avère être une étape clé du procédé.

En effet, c'est à partir de ce produit (3) que l'on pourra fabriquer bon nombre de monomères de l'invention.

En effet, dans le cas de schéma ci-dessus, le composé (3) est soumis à une étape de réduction pour conduire au produit (4) qui subit ensuite un greffage.

L'homme du métier comprendra aisément au vu de ce schéma qui comprend des opérations parfaitement classiques de chimie, que l'on pourra envisager d'autres étapes de « déprotection », par exemple l'hydrolyse de la fonction ester pour obtenir un acide carboxylique ou encore la réduction contrôlée de l'ester en aldéhyde.

On pourra également envisager d'autres étapes de greffage, par exemple la réaction du produit hydroxylé (4) avec un diisocyanate pour obtenir un photochrome à fonction réactive isocyanate ou encore la fonctionnalisation du produit (4) par réaction de transéthérification avec un éther énolique en présence d'acétate de mercure pour conduire à une fonction vinylique.

Par ailleurs, l'homme du métier comprendra aisément que l'on peut obtenir dans un schéma analogue à celui représenté ci-dessus un groupe époxy- par réaction du composé (4) avec de l'épichlorhydrine.

En ce qui concerne les monomères de l'invention comprenant deux groupements monovalents, ils pourront être obtenus en suivant le schéma ci-dessous ou un schéma dérivé de ce schéma aisément envisageable par l'homme du métier.

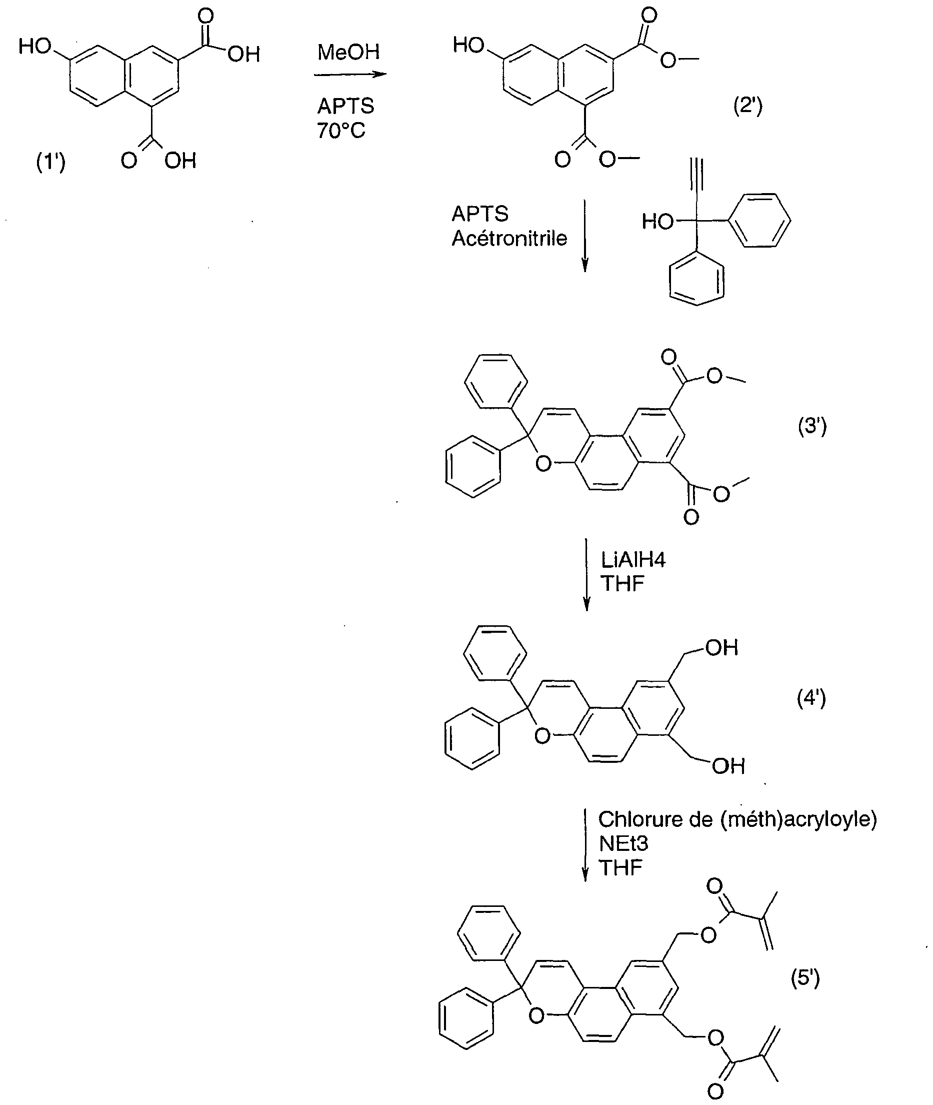

Comme dans le cas précédent, d'autres types de fonctions peuvent être envisagées, ainsi le groupe hydroxyle du composé (4') peut être transformé en amine ou l'ester (3') hydrolysé en acide carboxylique.

Selon son troisième aspect, l'invention concerne des polymères obtenus par copolymérisation d'un composé de l'invention avec un monomère.

Pratiquement tous les monomères existants peuvent être copolymérisés avec les monomères de l'invention. On pourrait également envisager la copolymérisation des monomères de l'invention avec d'autres monomères photochromes connus. Par exemple, celui décrit par Transition Optical dans WO 03/056390 porteur d'une fonction méthacrylate pourrait être copolymérisé avec le monomère de l'invention et du méthacrylate de méthyle pour fabriquer des verres organiques photochromes.

A titre d'exemples non limitatifs de comonomères que l'on peut envisager, on citera ceux porteurs d'une ou plusieurs fonctions hydroxyle, amino-, (méth)acrylate, vinylique, époxy-, isocyanato-, anhydride, acide, silane ou un mélange de différents monomères.

Le polymère photochromique obtenu présente une vitesse de décoloration et un rendement quantique proches de celui du photochrome faisant l'objet du premier aspect de l'invention.

Ainsi, il est possible de synthétiser des polymères hydrophobes pour des implants, pour des vernis, des polymères hydrophiles ou amphiphiles pour des crèmes etc.

Par exemple, le photochrome porteur d'une seule fonction hydroxyle (R₄ =-H), et R₁ à R₃ =H peut réagir avec un mélange de diol et de diisocyanate pour obtenir un polyuréthane photochromique.

Par exemple, le photochrome porteur d'une seule fonction acrylique (R₄= -C(=O)CH=CH₂), et R₁ à R₃ =H peut être copolymérisé avec l'acrylate de butyle et le styrène pour fabriquer un élément hydrophobe protecteur des UV lorsqu'il est soumis à la lumière du soleil.

Par exemple, le photochrome porteur d'une seule fonction méthacrylique R₄=-C(=O)CH=CH(CH₃), et R₁ à R₃ =H peut être copolymérisé avec le méthacrylate de 2-hydroxyéthyle ou l'acrylamide pour fabriquer un polymère soluble dans l'eau, entrant dans la composition de gels aqueux colorés sous UV.

On pourrait également envisager l'accrochage des monomères de l'invention sur des oligomères ou polymères existants. Par exemple, le monomère de type composé (4) portant à la fois un groupement R₄=-H et R₄= acrylate pourrait être greffé par estérification sur un polyacide et conduire à la réticulation de ce dernier par post-polymérisation radicalaire du groupe acryloxy. De nombreux oligomères ou polymères pourraient ainsi être modifiés chimiquement par les monomères photochromes de l'invention et devenir ainsi photosensibles.

Les possibilités sont très larges ; les exemples suivants ne sont pas limitatifs, ils ne sont qu'une illustration de l'invention.

### EXEMPLES

### Exemple 1 : Synthèse du 8-hydroxymethyl-3,3 diphényl-3H naphto [2,1]-pyrane (composé (4))

Cet exemple est donné en référence au 1^{er} schéma de synthèse.

### Etape 1 : Estérification du composé (1)

Dans un ballon à trois tubulures (250 ml), surmonté d'un condenseur, le composé (1) (7g) est dissout dans 80 ml de méthanol. La réaction d'estérification est catalysée par l'ajout d'acide para-toluènesulfonique (APTS, 0.48g) introduit sous azote. La réaction est menée à une température de 70°C et est laissée sous agitation au moins 12 heures.

A l'issue de ce délai, le méthanol est évaporé et la phase organique résiduelle est dissoute dans de l'acétate d'éthyle en vue d'extractions liquide/liquide (acétate d'éthyle/ eau saturée en carbonate de potassium) afin de purifier l'ester (composé (2)) attendu.

La phase organique résultante des différents lavages est ensuite séchée sur MgS04 puis filtrée. L'évaporation de l'acétate d'éthyle permet d'isoler de manière quantitative l'ester recherché (6 à 7 g).

### Etape 2 : Chroménisation du composé (2)

Dans un ballon à trois tubulures (100 ml), surmonté d'un condenseur, on introduit 1.56g de composé (2) avec 50 ml d'acétonitrile. Le milieu devient limpide dès la température de 50°C atteinte, moment où l'on ajoute, sous azote, 1.6g (1q/(2)) d'alcool propargylique et 0.122g d'APTS (0.08eq/(2)). Le milieu réactionnel est refroidi à température ambiante et on agite pendant deux jours à cette température.

Le composé (3) est simplement isolé par filtration puisque insoluble dans le milieu réactionnel. Il est purifié par lavages dans l'acétonitrile à 40°C suivis de filtrations. Le produit est obtenu avec un rendement de 55% sans purification supplémentaire.

### Etape 3 : Réduction de l'ester (3) pour atteindre (4) : 8-hydroxymethyl-3,3 diphényl-3H naphto [2,1]-pyrane

Dans un ballon à trois tubulures (100 ml), muni d'un bulleur, on introduit 0.2g de LiAlH₄ (1.41eq/(3)) dilué dans 15 ml de THF anhydre. On ajoute ensuite, sous azote et goutte à goutte les 1.5 g de (3) dissous dans 30ml de THF anhydre. Lors de l'addition aucun dégagement gazeux violent n'est observé. Le tout est agité au moins 12 heures à température ambiante.

Avant d'isoler le produit (4), l'excès de LiAlH₄ doit être neutralisé. Pour ce faire, on ajoute lentement 1.25 ml d'eau puis 12.5ml d'une solution d'acide sulfurique (H₂SO₄) à 10%. L'ajout d'éther dans le milieu fait apparaître deux phases. La phase organique extraite est lavée (eau saturée en NaCl) puis séchée avec MgS04 , filtrée et le solvant évaporé.

Le produit blanc obtenu avec un rendement quantitatif, correspond au composé (4) attendu.

### Exemple 2 : Fonctionnalisation avec le chlorure d'acryloyle : composé (5)

Dans un ballon à trois tubulures (100 ml), surmonté d'un condenseur, on introduit 1g de composé 4, 0.5ml de Et₃N (1.3eq/(4)) dissout dans 30ml de THF anhydre. L'addition du chlorure est faite goutte à goutte, sous azote, à température ambiante, sans observer d'élévation de température importante. La réaction est laissée à température ambiante et sous agitation pendant au moins 12 heures.

A l'issue de cette période, le solvant est évaporé. Le résidu est dilué dans le dichlorométhane puis extrait par lavages successifs avec de l'eau saturée en carbonate de potassium. La phase organique extraite est séchée avec MgS04 , filtrée sur silice et le solvant évaporé.

La molécule (5) est isolée avec un rendement massique de 60%.

Le tableau ci-dessous rassemble les résultats obtenus en terme de rendement quantique, vitesse de décoloration et résistance à la fatigue du produit obtenu, en comparaison avec différents produits de la littérature.

| Référence | Couleur sous forme excitée | Rendement quantique | Vitesse de décoloration | Résistance à la fatigue |
|---|---|---|---|---|
| **diphényl-naphtopyrane (invention)** | **jaune-orange** | **0.3-0.4** | **<200** | **bonne** |
| **naphtopyranes (**US6113814**)** | **jaune-vert-bleu** | **0.2-0.7 (UV/90s)** | **40-270** | **bonne** |
| **dinaphto-pyranes (**US5464567**)** | **bleu-violet** | **0.2 (UV/5s)** | **150-600** | **bonne** |
| **indeno-naphto-pyrane (WO03056390,** WO9614596**, matrice polymère)** | **jaune-brun** | **0.3-0.7 (UV/15min)** | **50-100** | **moyenne** |

| | | | | |
|---|---|---|---|---|
| *Rendement quantique : efficacité à absorber les rayons UV pour provoquer l'ouverture du cycle à 22°C, sur 5 secondes d'irradiation.* *Vitesse de décoloration : vitesse à laquelle la couleur disparaît lorsqu'on arrête les UV; ici, cette vitesse est représentée par le temps au bout duquel le rendement quantique tombe à 50% (en secondes) ; dans certains cas, elle a été mesurée dans une matrice polymère qui fait passer un t*_{*1*/}*₂ de 50 à 100s généralement.* *Résistance à la fatigue : qualité du photochrome à effectuer de nombreux cycles coloration*/*décoloration; une bonne résistance à la fatigue correspond à la perte de 5% de rendement après une cinquantaine de cycles.* | | | | |

### Exemple 3 : Fonctionnalisation avec le chlorure de méthacryrloyle

La synthèse est identique à celle de l'exemple 2, sauf que le chlorure d'acryloyle est remplacé par le chlorure de méthacryloyle.

### Exemple 4 : synthèse d'un polyuréthane à partir du produit (4) de l'exemple 1 et d'un diisocyanate

Dans un réacteur de un litre surmonté d'un condenseur, on introduit 400mL de méthyéthylcétone (MEK) séchée et on dissout 50g de polyoxyde d'éthylène (POE) dihydroxyle séché (Mn=200g/mol, soit 0.05 mol de fonctions OH) et 26.2g de 4,4'-méthylènebiscyclohexyl diisocyanate (M=262g/mol, soit 0,1 mol de fonctions NCO) sous courant d'azote. Le réacteur est chauffé par une double enveloppe au reflux du solvant (70°C) puis on ajoute une quantité catalytique de dibutyldilaurate d'étain.

Lorsque la consommation en isocyanate n'évolue plus par suivi par spectroscopie infrarouge, on ajoute la molécule photochromique 1 (19.4g, soit 0.05 mol) pour terminer les chaînes.

Le polymère obtenu est récupéré par précipitation dans un mélange d'essences avec un excellent rendement (>90%). Mis en solution dans l'eau, il développe réversiblement la coloration orange en quelques secondes sous UV.

### Exemple 5 : Synthèse d'un polymère acrylique hydrophobe à partir du composé (5), de styrène et d'acrylate de butyle

Dans un ballon de 250mL surmonté d'un condenseur, on introduit 100mL de tétrahydrofurane (THF) distillé et on dissout 70g de styrène distillé, 30g d'acrylate de butyle distillé, 0.2g de la molécule (5) issue de l'exemple 2, puis 0.2g de 2,2'-azobisisobutyronitrile. On fait barboter l'azote pour dégazer le milieu réactionnel puis on chauffe le ballon à 60°C pendant 4h.

Le polymère est récupéré par précipitation dans l'éthanol avec un rendement massique de 80%.

Mis sous forme de revêtement sur une plaque de verre, le polymère développe une coloration jaune orangée immédiate sous UV et se décolore en 20 secondes à l'abri de la lumière.

### Exemple 6 : Synthèse d'un polymère méthacrylique hydrophile à partir du produit de l'exemple 3 et de méthacrylate d'hydroxyéthyle

Dans un ballon de 250mL surmonté d'un condenseur, on introduit 100g de méthacrylate d'hydroxyéthyle distillé, 0.5g de diméthacrylate d'éthylèneglycol, 0.2g de la molécule (5) issue de l'exemple 3, puis 0.2g de 2,2'-azobisisobutyronitrile. On fait barboter l'azote pour dégazer le milieu réactionnel visqueux puis on coule la solution obtenue dans un moule, un petit tube par exemple. Ce moule est porté à 50°C pendant 12h puis 60°C pendant 4h et enfin 80°C pendant 4h.

Le polymère sous forme de tige est recuit à l'étuve pendant 12h à 80°C. Lorsqu'on le trempe dans l'eau, il se gonfle et développe réversiblement la couleur jaune lorsqu'on le soumet à la lumière du soleil.

### Exemple 7 : Photopolymérisation du produit de l'exemple 2 et de l'acrylate de benzyle

Dans un mélange d'acrylate de benzyle (99%), de diacrylate d'hexanediol (HDDA, 1%) contenant 0.2% de 2,2-diméthoxy-1,2-diphénylethan-1-one ou 1-hydroxy cyclohexyl phenyl cétone , on ajoute 0.5% en poids du monomère de l'exemple 2. Après dégazage par barbotage d'azote pendant 15 minutes, le mélange est placé dans un moule en verre et irradié avec des lampes à ultraviolets pendant 300 à 1200 secondes en fonction de la lampe.

Le film obtenu développe une coloration jaune orangée immédiate sous nouvelle stimulation UV et se décolore rapidement à l'abri de la lumière.

## Revendications

1. Composé du type 3,3-diaryl-3H-naphto[2,1-b]pyrane substitué polymérisable **caractérisé en ce qu'**il répond à la formule (I) : 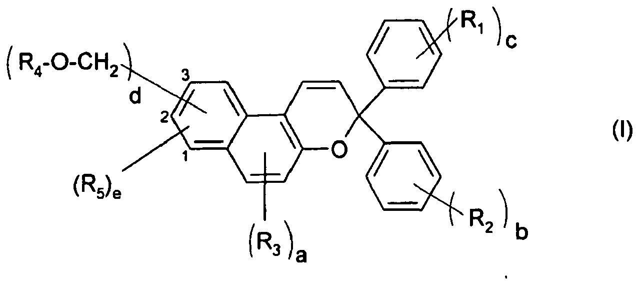 dans laquelle
• R₁, R₂ et R₃ sont tels qu'ils sont identiques ou différents et représentent indépendamment :
- un hydrogène,
- un halogène,
- un groupement hydroxy ou hydroxyalkyle en C1 à C15 ou
- un groupement alkyle linéaire ou ramifié en C1 à C15 a, b et c étant indépendamment compris entre 0 et 5 ;
• R₄ est lié au motif naphtalène en position 1, 2 ou 3 via une liaison -CH₂-O- et choisi parmi :
- les groupes divalents suivants :
CH₂=CH-C(=O)-, CH₂=C(CH₃)-C(=O)-, -(CH₂)-OC(=O)CH=CH₂, -(CH₂)-OC(=O)C(CH₃)=CH₂, -(CH₂)_{n'}-CH=CH2_{,} -(CH₂)-O-(CH)_{n'}-CH=CH₂, -(CH₂)-O-CH=CH₂, n' étant compris entre 0 et 15,
et, dans ce cas, le(s) groupe(s) R₅ est (sont) H, (CₙH₂ₙ)-CH₃, (CₙH₂ₙ)-OH, n étant compris entre 1 et 15, et
d est alors compris entre 1 et 3 ;
- les groupes monovalents suivants :
l'hydrogène, (CH₂)-NH₂,-(CₚH₂ₚ) - [CH-CH₂-O]cycle,
-(CH₂)-COOH, -(CH₂)Si(CₘH₂ₘ)₂-H, -(CH₂)-Si-CH=CH₂, -CH₂)Si(O-CₘH₂ₘ)₃,
-C(=O)NH-R-N=C=O, avec R = (CₙH₂ₙ), (CₙH₂ₙ₋₂), aryle en C₅ à C₂₀,
(CₙH₂ₙ₋₂)-CH₂-(CₙH₂ₙ₋₂) et Aryle-CH₂-Aryle, n et m étant compris entre 1 et 15 et
p étant compris entre 0 et 15 ,
et dans ce cas le groupe R₅, s'il y en a un, est H ou un alkyle en C₁-C₁₅, et
d est alors égal à 2 ou 3 ;
• R₅ est tel que défini ci-dessus et est lié au motif naphtalène en position 1, 2 ou 3,
et e est un entier compris entre 0 et 2 et tel que d+ e = 3.

2. Composé selon la revendication 1, **caractérisé en ce que** les groupements R₁ et R₂ sont choisis indépendamment dans le groupe constitué de l'hydrogène, du méthyle et du fluor et **en ce que** R₅ et R₃ sont de l'hydrogène.

3. Composé selon l'une des revendications 1 ou 2, **caractérisé en ce que** au moins deux des positions 1, 2 et 3 du motif naphtalène portent un groupement R₄-O-CH₂- avec R₄ choisi indépendamment dans le groupe constitué de l'hydrogène, -(CH₂)-NH₂, -(CH₂)-COOH, -(CₚH₂ₚ)-[CH-CH₂-O]cycle, (CH₂)- Si(CₘH₂ₘ)-H, (CH₂)- Si(O-CₘH₂ₘ)₃, -C(=O)NH-R-N=C=O, avec R = (CH₂) ou (CₙH₂ₙ₋₂) ou -(CₙH₂ₙ-₂)-CH₂-(CₙH₂ₙ₋₂) ou aryle en C₅ à C₂₀, et Aryle-CH₂-Aryle, avec n et m étant compris entre 1 et 15 et p compris entre 0 et 15 ; le groupe R₅, s'il y en a un, est H ou un alkyle en C₁-C₅.

4. Composé selon l'une des revendications 1 ou 2, **caractérisé en ce que** les trois substituants portés en position 1, 2 et 3 du motif naphtalène sont de la forme R₄-O-CH₂- avec R₄ choisi indépendamment dans le groupe constitué de l'hydrogène, , -((CH₂)- NH₂, -((CH₂)- COOH, -(CₚH₂ₚ)-[CH-CH₂-O]cycle, (CH₂)- Si(CₘH₂ₘ)-H, (CH₂)- Si(O-CₘH₂ₘ)₃, -C(=O)NH-R-N=C=O, avec R = (CH₂) ou (CₙH₂ₙ₋₂) ou -(CₙH₂ₙ₋₂)-CH₂-(CₙH₂ₙ₋₂) ou aryle en C₅ à C₂₀, et Aryle-CH₂-Aryle, avec n et m étant compris entre 1 et 15 et p compris entre 0 et 15.

5. Composé selon la revendication 1, **caractérisé en ce qu'**il répond à la formule : 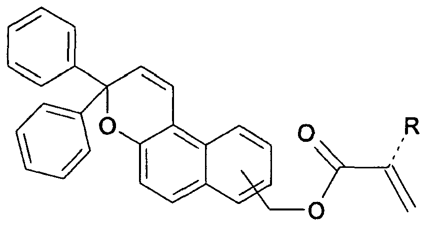 avec R = H ou CH₃

6. Procédé de synthèse d'un composé selon l'une des revendications 1 à 5, **caractérisé en ce qu'**il comprend une étape de cyclisation, dite étape de chroménisation, au cours de laquelle on précipite dans le milieu réactionnel le produit intermédiaire répondant à la formule II ci-dessous : 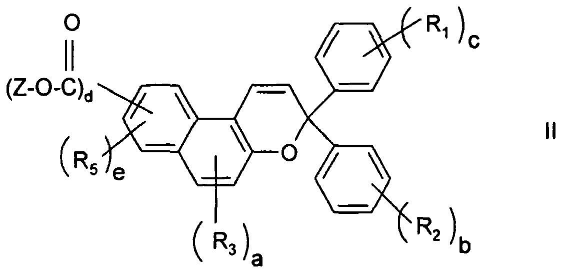 dans laquelle le groupement Z est soit l'hydrogène, soit un groupement alkyle CₙH₂ₙ₊₁ avec n= 1 à 15, soit un précurseur, éventuellement protégé, des groupements R₄, les groupements R₁, R₂, R₃, R₄ et R₅ ainsi que a, b, c, d et e étant tels que définis dans les revendications précédentes.

7. Utilisation d'un composé selon l'une des revendications 1 à 5 dans la préparation d'un polymère, d'un copolymère ou d'un oligomère à propriété photochrome ou photosensible.

8. Utilisation selon la revendication 7 **caractérisée en ce que** ledit composé est utilisé en tant que monomère ou comonomère dans une réaction de polymérisation ou de copolymérisation destinée à produire un polymère ou un copolymère à propriété photochrome ou photosensible.

9. Utilisation selon la revendication 8 **caractérisé en ce que** ledit composé est utilisé pour modifier chimiquement un oligomère ou un polymère et le rendre photosensible.

## Claims

1. Compound of the polymerisable substituted 3,3-diaryl-3H-naphtho[2,1-b]pyrane type, **characterised in that** it corresponds to formula (I): 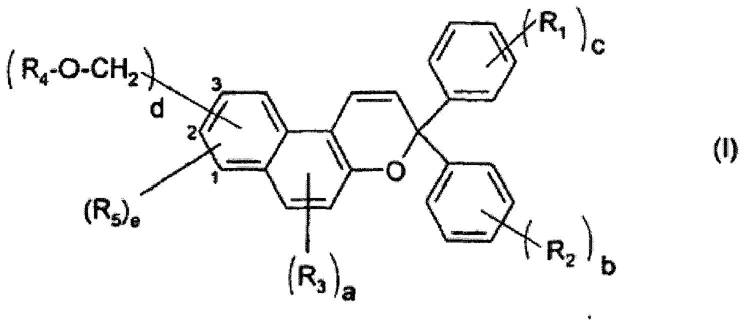 in which
• R₁, R₂ and R₃, identical or different, independently represent:
- a hydrogen
- a halogen
- a hydroxy or a C₁ to C₁₅ hydroxyalkyl group or
- a C₁ to C₁₅ linear or branched alkyl group a, b and c independently lying between 0 and 5;
• R₄ is linked to the naphthalene unit at position 1, 2 or 3 via a -CH₂-O-bond and selected from:
- the following divalent groups:
CH₂=CH-C(=O)-, CH₂=C(CH₃)-C(=O)-, -(CH₂)-OC(=O)CH=CH₂,
-(CH₂)-OC(=O)C(CH₃)=CH₂, -(CH₂)_{n'}-CH=CH₂, -(CH₂)-O-(CH)_{n'}-CH=CH₂,
-(CH₂)-O-CH=CH₂, n' lying between 0 and 15,
and, in this case the group(s) R₅ is(are) H, (CₙH₂ₙ)-CH₃, (CₙH₂ₙ)-OH, n lying between 1 and 15, and
d then lies between 1 and 3;
- the following monovalent groups:
hydrogen, (CH₂)-NH₂,-(CₚH₂ₚ)- [CH-CH₂-O]ring,
-(CH₂)-COOH, -(CH₂)Si(CₘH₂ₘ)₂-H, -(CH₂)-Si-CH=CH₂,
-(CH₂)Si(O-CₘH₂ₘ)₃,-C(=O)NH-R-N=C=O, with R = (CₙH₂ₙ), (CₙH₂ₙ₋₂),
C₅ to C₂₀ aryl, (CₙH₂ₙ₋₂)-CH₂-CₙH₂ₙ₋₂) and aryl-CH₂-aryl, n and m lying between 1 and 15 and p lying between 0 and 15,
and in this case the group R₅, if there is one, is H or a C₁ to C₁₅ alkyl, and
d is then equal to 2 or 3;
• R₅ is as defined above and is linked to the naphthalene unit at position 1, 2 or 3,
and e is an integer comprised between 0 and 2 and such that d+e=3.

2. Compound according to claim 1, **characterised in that** the groups R₁ and R₂ are selected independently from the group consisting of hydrogen, methyl and fluorine and **in that** R₅ and R₃ are hydrogen.

3. Compound according to one of claims 1 or 2, **characterised in that** at least two of positions 1, 2 and 3 of the naphthalene unit carry a group R₄-O-CH₂ with R₄ selected independently from the group consisting of hydrogen, (CH₂)-NH₂, -(CH₂)-COOH, -(CₚH₂ₚ)-[CH-CH₂-O]ring, (CH₂)-Si(CₘH₂ₘ)-H, (CH₂)-Si(O-CₘH₂ₘ)₃, -C(=O)NH-R-N=C=O, with R = (CH₂) or (CₙH₂ₙ₋₂) or -(CₙH₂ₙ₋₂)-CH₂-(CₙH₂ₙ₋₂) or C₅ to C₂₀ aryl, and aryl-CH₂-aryl, with n and m lying between 1 and 15 and p lying between 0 and 15; the group R₅, if there is one, is H or a C₁ to C₅ alkyl.

4. Compound according to one of claims 1 or 2, **characterised in that** the three substituents carried at positions 1, 2 and 3 of the naphthalene unit are of the form R₄-O-CH₂- with R₄ selected independently from the group consisting of hydrogen, -(CH₂)-NH₂, -(CH₂)-COOH, -(CₚH₂p)-[CH-CH₂-O]ring, (CH₂)-Si(CₘH₂ₘ)-H, (CH₂)-Si(O-CₘH₂ₘ)₃, -C(=O)NH-R-N=C=O, with R = (CH₂) or (CₙH₂ₙ₋₂) or -(CₙH₂ₙ₋₂)-CH₂-(CₙH₂ₙ₋₂),
or C₅ to C₂₀ aryl and aryl-CH₂-aryl, with n and m lying between 1 and 15 and p lying between 0 and 15;

5. Compound according to claim 1, **characterised in that** it corresponds to the formula: 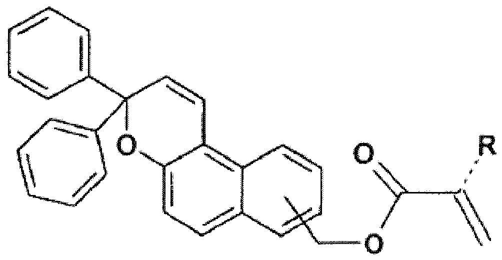 with R = H or CH₃

6. Process for synthesising a compound according to one of claims 1 to 5, **characterised in that** it comprises a step of cyclisation, called chromenisation step, during which the intermediate product corresponding to formula II below is precipitated in the reaction medium: 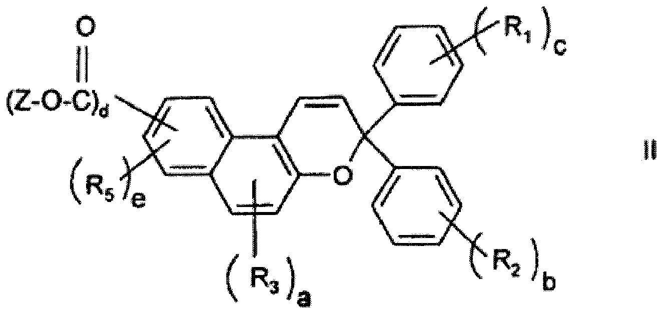 in which the group Z is either hydrogen or an alkyl group CₙH₂ₙ₊₁ with n=1 to 15 or a precursor, optionally protected, of the groups R₄, the groups R₁ R₂, R₃, R₄ and R₅ as well as a, b, c, d and e being as defined in the preceding claims.

7. Use of a compound according to one of claims 1 to 5 in the preparation of a polymer, a copolymer or an oligomer with a photochromic or photosensitive property.

8. Use according to claim 7, **characterised in that** said compound is used as a monomer or comonomer in a polymerisation or copolymerisation reaction intended to produce a polymer or a copolymer with a photochromic or photosensitive property.

9. Use according to claim 8, **characterised in that** said compound is used to chemically modify an oligomer or a polymer and render it photosensitive.

## Patentansprüche

1. Verbindung vom Typ polymerisierbares, substituiertes 3,3-Diaryl-3H-naphtho[2,1-b]pyran,
**dadurch gekennzeichnet, dass** sie der Formel (I) entspricht: 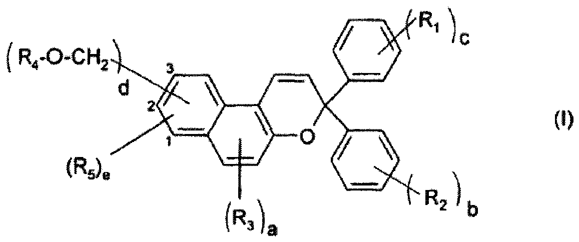 worin
• R₁, R₂ und R₃ derart sind, dass sie identisch oder verschieden sind und unabhängig voneinander darstellen:
- Wasserstoff,
- Halogen,
- eine Hydroxy- oder C₁-C₁₅-Hydroxyalkyl - Gruppe,
- eine lineare oder verzweigte C₁-C₁₅ - Alkyl-Gruppe,
wobei a, b und c unabhängig voneinander zwischen 0 und 5 betragen;
• R₄ mit der Naphthalin-Einheit in 1-, 2- oder 3-Stellung über eine -CH₂-O-Bindung verbunden und ausgewählt ist aus:
- den folgenden zweiwertigen Gruppen:
CH₂=CH-C(=O)-, CH₂=C(CH₃)-C(=O)-, -(CH₂)-OC(=O)CH=CH₂, -(CH₂)-OC(=O)C(CH₃)=CH₂, -(CH₂)_{n'}-CH=CH₂, -(CH₂)-O-(CH)_{n'}-CH=CH₂,-(CH₂)-O-CH=CH₂, wobei n' zwischen 0 und 15 beträgt, und dabei die R₅-Gruppe(n) H, (CₙH₂ₙ)-CH₃, (CₙH₂ₙ)-OH ist/ sind,
wobei n zwischen 1 und 15 beträgt, und
d dann zwischen 1 und 3 beträgt;
- den folgenden einwertigen Gruppen:
Wasserstoff, (CH₂)-NH₂, -(CₚH₂ₚ) - [CH-CH₂-O] Cyclus,
-(CH₂)-COOH, -(CH₂)Si(CₘH₂ₘ)₂-H, -(CH₂)-Si-CH=CH₂, -(CH₂)Si(O-CₘH₂ₘ)₃, -C(=O)NH-R-N=C=O, mit R = (CₙH₂ₙ),
(CₙH₂ₙ₋₂), C₅-C₂₀-Aryl, (CₙH₂ₙ₋₂)-CH₂-(CₙH₂ₙ₋₂) und Aryl-CH₂-Aryl,
wobei n und m zwischen 1 und 15 betragen und p zwischen 0 und 15 beträgt,
und dabei die R₅-Gruppe, falls vorhanden, H oder ein C₁-C₁₅-Alkyl ist und d dann gleich 2 oder 3 ist;
• R₅ wie oben definiert ist und mit der Naphthalin-Einheit in 1-, 2-oder 3-Stellung verbunden ist,
und e ein Ganzes zwischen 0 und 2 beträgt und derart ist, dass d+e=3ist.

2. Verbindung nach Anspruch 1,
**dadurch gekennzeichnet, dass** die R₁- und R₂-Gruppen unabhängig voneinander ausgewählt sind aus der Gruppe umfassend Wasserstoff, Methyl und Fluor, und dass R₅ und R₃ Wasserstoff sind.

3. Verbindung nach einem der Ansprüche 1 und 2,
**dadurch gekennzeichnet, dass** zumindest zwei der 1-, 2-, und 3-Stellungen der Naphthalin-Einheit eine R₄-O-CH₂-Gruppe tragen, wobei R₄ unabhängig ausgewählt ist aus der Gruppe umfassend Wasserstoff, -(CH₂)-NH₂, -(CH₂)-COOH, -(CₚH₂ₚ) - [CH-CH₂-O] Cyclus, (CH₂)- Si(CₘH₂ₘ)-H, (CH₂)- Si(O-CₘH₂ₘ)₃, -C(=O)NH-R-N=C=O, mit R = (CH₂) oder (CₙH₂ₙ₋₂) oder -(CₙH₂ₙ₋₂)-CH₂-(CₙH₂ₙ₋₂) oder C₅-C₂₀-Aryl und Aryl-CH₂-Aryl, wobei n und m zwischen 1 und 15 betragen und p zwischen 0 und 15 beträgt, und dabei die R₅-Gruppe, falls vorhanden, H oder ein C₁-C₅-Alkyl ist.

4. Verbindung nach einem der Ansprüche 1 und 2,
**dadurch gekennzeichnet, dass** die drei in 1-, 2- und 3-Stellung getragenen Substituenten der Naphtalin-Einheit die Form R₄-O-CH₂- haben, wobei R₄ unabhängig ausgewählt ist aus der Gruppe umfassend Wasserstoff, -(CH₂)-NH₂, -(CH₂)-COOH, -(CₚH₂ₚ) - [CH-CH₂-O] Cyclus, (CH₂)- Si(CmH2m)-H, (CH₂)- Si(O-CₘH₂ₘ)₃, -C(=O)NH-R-N=C=O, mit R = (CH₂) oder (CₙH₂ₙ₋₂) oder -(CₙH₂ₙ₋₂)-CH₂-(CₙH₂ₙ₋₂) oder C₅-C₂₀-Aryl und Aryl-CH₂-Aryl, wobei n und m zwischen 1 und 15 betragen und p zwischen 0 und 15 beträgt.

5. Verbindung nach Anspruch 1,
**dadurch gekennzeichnet, dass** sie der Formel entspricht: 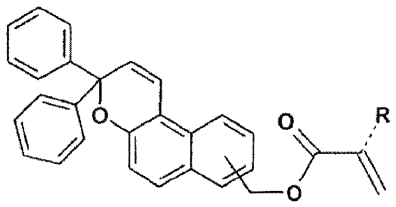 mit R = H oder CH₃.

6. Verfahren zur Synthese einer Verbindung nach einem der Ansprüche 1 bis 5,
**dadurch gekennzeichnet, dass** es einen Schritt der Cyclisierung, die sogenannte Chromenisation, umfasst, bei der in dem Reaktionsmedium das der nachfolgenden Formel II entsprechende Zwischenprodukt ausgefällt wird: 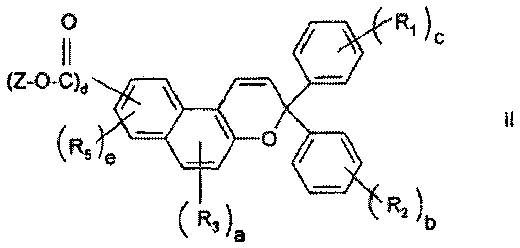 worin die Z-Gruppe entweder Wasserstoff oder eine CₙH₂ₙ₊₁-Alkyl-Gruppe mit n = 1 bis 15 oder ein gegebenenfalls geschützter Vorläufer der R₄-Gruppen ist, wobei die R₁-, R₂-, R₃-, R₄- und R₅-Gruppen sowie a, b, c, d und e wie in den vorangehenden Ansprüchen definiert sind.

7. Verwendung einer Verbindung nach einem der Ansprüche 1 bis 5 bei der Herstellung eines Polymers, eines Copolymers oder eines Oligomers mit photochromer oder lichtempfindlicher Eigenschaft.

8. Verwendung nach Anspruch 7,
**dadurch gekennzeichnet, dass** die Verbindung als Monomer oder Comonomer bei einer Polymerisations- oder Copolymerisationsreaktion verwendet wird, die dazu bestimmt ist, ein Polymer oder ein Copolymer mit photochromer oder lichtempfindlicher Eigenschaft zu erzeugen.

9. Verwendung nach Anspruch 8,
**dadurch gekennzeichnet, dass** die Verbindung dazu verwendet wird, ein Oligomer oder ein Polymer chemisch zu verändern und es lichtempfindlich zu machen.
